# EUROPEAN PATENT APPLICATION

(11) **EP 4 628 076 A1**
(43) Date of publication of application: **08.10.2025**
(21) Application number: 23897613.8
(22) Date of filing: 21.11.2023
(51) Int. Cl.: A61K 31/381, A61K 9/70, A61K 47/10, A61K 47/32, A61K 47/34, A61P 25/00, A61P 25/16

(54) **ROTIGOTINE-CONTAINING PATCH AND METHOD FOR IMPROVING STABILITY OF ROTIGOTINE**

(30) Priority: 01.12.2022 JP 2022192802
(71) Applicant: Hisamitsu Pharmaceutical Co., Inc., Tosu-shi, Saga 841-0017 (JP)
(72) Inventor: KAWAI Kimiko, Tsukuba-shi, Ibaraki 305-0856 (JP); SHINODA Tomohiro, Tsukuba-shi, Ibaraki 305-0856 (JP); KUROKAWA Takao, Tsukuba-shi, Ibaraki 305-0856 (JP); TAKAMI Atsuro, Tsukuba-shi, Ibaraki 305-0856 (JP)
(74) Representative: Grünecker Patent- und Rechtsanwälte PartG mbB
(86) International application number: PCT/JP2023/041779
(87) International publication number: WO 2024/116965

(57) **Abstract**

A rotigotine-containing patch including a backing layer and an adhesive agent layer, wherein the adhesive agent layer comprises an adhesive base agent, propyl gallate, and at least one selected from the group consisting of rotigotine and a pharmaceutically acceptable salt of rotigotine.

## Description

### [Technical Field]

The present invention relates to a rotigotine-containing patch (adhesive patch) and a method for improving stability of rotigotine, and more specifically, to a patch comprising rotigotine and/or a pharmaceutically acceptable salt thereof, and a method for improving rotigotine stability in such a patch.

### [Background Art]

Rotigotine is the international nonproprietary name of the compound (-)-5,6,7,8-tetrahydro-6-[propyl-[2-(2-thienyl)ethyl]-amino]-1-naphthalenol; it is described in International Application Japanese-Phase Publication No. 2011-504902 (Patent Literature 1) that there exist type I and type II crystalline polymorphs thereof. Rotigotine is a D1/D2/D3 dopamine receptor agonist and is mainly used in the treatment of symptoms of Parkinson's disease and restless legs syndrome.

As a pharmaceutical preparation for administration of rotigotine, for example, "Neupro (registered trademark) Patch" is commercially available in Japan and overseas. Also, International Application Japanese-Phase Publication No. 2002-509878 (Patent Literature 2) describes a transdermal therapeutic system including a backing layer inert to the components of a matrix and a self-adhesive matrix layer comprising rotigotine, wherein the matrix has a rotigotine solubility of 5% (w/w) or more and comprises, as a base, a water-insoluble acrylate-based or silicone-based polymer adhesive.

Furthermore, International Application Japanese-Phase Publication No. 2015-503541 (Patent Literature 3) describes a transdermal therapeutic system including a backing layer impermeable to an active substance and a matrix layer comprising a pressure-sensitive adhesive, a drug, and particles of crosslinked polyvinylpyrrolidone, wherein rotigotine is described as the drug and a silicone polymer is described as the pressure-sensitive adhesive. Further, for example, Japanese Unexamined Patent Application Publication No. 2014-177428 (Patent Literature 4) describes a transdermal absorption adhesive preparation including a support, a rubber-based adhesive base agent comprising a rosin-based resin and a rubber-based adhesive component, and a drug-containing layer comprising rotigotine or a pharmaceutically acceptable salt thereof, and Japanese Unexamined Patent Application Publication No. 2013-079220 (Patent Literature 5) describes a transdermal absorption adhesive preparation including a support, and a drug-containing layer comprising a rubber-based adhesive base agent, rotigotine or a salt thereof, and a production inhibitor of degradation products of rotigotine.

Furthermore, for example, International Application Japanese-Phase Publication No. 2017-515871 (Patent Literature 6) describes a method for producing a transdermal absorption preparation, in which rotigotine and an antioxidant such as butylhydroxytoluene are mixed at a specific weight ratio in order to prevent rotigotine crystal precipitation. In addition, as a patch comprising the antioxidant, for example, Domestic Re-Publication of PCT International Application No. 2017-073516 (Patent Literature 7) and Domestic Re-Publication of PCT International Application No. 2018-198925 (Patent Literature 8) describe that an adhesive agent layer of a patch comprising fentanyl or butorphanol as a drug comprises an antioxidant having a sulfur atom in the molecule.

### [Citation List]

### [Patent Literature]

[PTL 1] International Application Japanese-Phase Publication No. 2011-504902
[PTL 2] International Application Japanese-Phase Publication No. 2002-509878
[PTL 3] International Application Japanese-Phase Publication No. 2015-503541
[PTL 4] Japanese Unexamined Patent Application Publication No. 2014-177428
[PTL 5] Japanese Unexamined Patent Application Publication No. 2013-079220
[PTL 6] International Application Japanese-Phase Publication No. 2017-515871
[PTL 7] Domestic Re-Publication of PCT International Application No. 2017-073516
[PTL 8] Domestic Re-Publication of PCT International Application No. 2018-198925

### [Summary of Invention]

### [Technical Problem]

However, as a result of further studies on the rotigotine-containing patch comprising rotigotine and/or a pharmaceutically acceptable salt thereof, the present inventors have found that the stability of rotigotine and/or a pharmaceutically acceptable salt thereof is not sufficient under storage conditions, for example, severe storage conditions such as high temperature, and rotigotine and/or a pharmaceutically acceptable salt thereof may be degraded to generate degradation products thereof (rotigotine degradation products). The present inventors have found that a further high level of stability over time is required because there is a risk that the desired efficacy of rotigotine and/or a pharmaceutically acceptable salt thereof may not be expected if a large amount of rotigotine degradation products are generated.

The present invention has been made in view of the above problems, and an object thereof is to provide a rotigotine-containing patch and a method for improving rotigotine stability, which are particularly excellent in the stability over time of rotigotine and/or a pharmaceutically acceptable salt thereof.

### [Solution to Problem]

As a result of intensive studies directed toward achieving the above object, the present inventors have found that in a rotigotine-containing patch, which is a patch including a backing layer and an adhesive agent layer and in which the adhesive agent layer comprises at least one selected from the group consisting of rotigotine and a pharmaceutically acceptable salt thereof (in the present specification, sometimes referred to as "rotigotine and/or a pharmaceutically acceptable salt thereof") and an adhesive base agent, by further comprising propyl gallate in the adhesive agent layer, the generation of rotigotine degradation products can be suppressed at a high level even during storage under severe conditions, so that it is possible to obtain a rotigotine-containing patch that is particularly excellent in the stability over time of rotigotine and/or a pharmaceutically acceptable salt thereof, thereby completing the present invention. Aspects of the present invention obtained by such findings are as follows.
[1] A rotigotine-containing patch including a backing layer and an adhesive agent layer, wherein the adhesive agent layer comprises an adhesive base agent, propyl gallate, and at least one selected from the group consisting of rotigotine and a pharmaceutically acceptable salt of rotigotine.
[2] The rotigotine-containing patch according to [1], wherein a content of propyl gallate in the adhesive agent layer is 0.01 to 1% by mass relative to a total mass of the adhesive agent layer.
[3] The rotigotine-containing patch according to [1] or [2], wherein the adhesive base agent is at least one selected from the group consisting of a rubber-based adhesive base agent, an acrylic-based adhesive base agent, and a silicone-based adhesive base agent.
[4] The rotigotine-containing patch according to any one of [1] to [3], wherein a content of the at least one selected from the group consisting of rotigotine and a pharmaceutically acceptable salt of rotigotine in the adhesive agent layer is 5 to 15% by mass relative to the total mass of the adhesive agent layer in terms of rotigotine free form.
[5] A method for improving stability of at least one selected from the group consisting of rotigotine and a pharmaceutically acceptable salt of rotigotine in a rotigotine-containing patch including a backing layer and an adhesive agent layer, wherein the adhesive agent layer comprises an adhesive base agent and at least one selected from the group consisting of rotigotine and a pharmaceutically acceptable salt of rotigotine, the method including:
   a step of further comprising propyl gallate in the adhesive agent layer.
[6] The method for improving rotigotine stability according to [5], wherein propyl gallate is comprised in the adhesive agent layer such that a content thereof relative to the total mass of the adhesive agent layer is 0.01 to 1% by mass.
[7] The method for improving rotigotine stability according to [5] or [6], wherein the adhesive base agent is at least one selected from the group consisting of a rubber-based adhesive base agent, an acrylic-based adhesive base agent, and a silicone-based adhesive base agent.
[8] The method for improving rotigotine stability according to any one of [5] to [7], wherein the content of the at least one selected from the group consisting of rotigotine and a pharmaceutically acceptable salt of rotigotine is 5 to 15% by mass relative to the total mass of the adhesive agent layer in terms of rotigotine free form.

### [Advantageous Effects of Invention]

According to the present invention, it is possible to provide a rotigotine-containing patch and a method for improving rotigotine stability, which are particularly excellent in the stability over time of rotigotine and/or a pharmaceutically acceptable salt thereof.

### [Description of Embodiments]

Hereinafter, the present invention will be described in detail with reference to preferred embodiments thereof.

### [Rotigotine-Containing Patch]

The rotigotine-containing patch of the present invention includes a backing layer and an adhesive agent layer, wherein the adhesive agent layer comprises an adhesive base agent, propyl gallate, and at least one selected from the group consisting of rotigotine and a pharmaceutically acceptable salt of rotigotine.

The rotigotine-containing patch of the present invention includes a backing layer and an adhesive agent layer. The backing layer is not particularly limited as long as it can support the adhesive agent layer described later, and a known backing layer of a patch can be appropriately adopted. Examples of the material of the backing layer according to the present invention include polyolefins such as polyethylene and polypropylene; ethylene-vinyl acetate copolymers, vinyl acetate-vinyl chloride copolymers, polyvinyl chloride, and the like; polyamides such as nylon; polyesters such as polyethylene terephthalate (PET), polybutylene terephthalate, and polyethylene naphthalate; cellulose derivatives; synthetic resins such as polyurethane; and metals such as aluminum. Among these, polyester and polyethylene terephthalate are preferable from the viewpoints of drug non-adsorption and drug impermeability. Examples of the form of the backing layer include films; sheet-shaped objects such as sheets, sheet-shaped porous bodies, and sheet-shaped foams; fabrics such as woven fabrics, knitted fabrics, and nonwoven fabrics; foils; and laminates thereof. Further, the thickness of the backing layer is not particularly limited, but is preferably in the range of 5 to 1000 µm from the viewpoints of workability and ease of production when the patch is applied.

The rotigotine-containing patch of the present invention may further include a release liner on a surface of the adhesive agent layer opposite to the backing layer. Examples of such a release liner include films and sheets made of materials, namely polyolefins such as polyethylene and polypropylene; ethylene-vinyl acetate copolymers, vinyl acetate-vinyl chloride copolymers, polyvinyl chloride, and the like; polyamides such as nylon; polyesters such as polyethylene terephthalate; cellulose derivatives; synthetic resins such as polyurethane; and aluminum and paper, and laminates thereof. These release liners are preferably subjected to a release treatment such as a silicone compound coating or a fluorine-containing compound coating on the surface thereof that comes into contact with the adhesive agent layer so that they can be easily peeled off from the adhesive agent layer.

### <Rotigotine and Pharmaceutically Acceptable Salt Thereof>

The adhesive agent layer according to the present invention comprises, as a drug, at least one selected from the group consisting of rotigotine and a pharmaceutically acceptable salt thereof (rotigotine and/or a pharmaceutically acceptable salt thereof). In the present invention, the form of rotigotine comprised in the adhesive agent layer may be a free form or a pharmaceutically acceptable salt thereof, and a pharmaceutically acceptable salt of rotigotine may be desalted during production and/or in the produced preparation to be in a free form, and may be one or a mixture of two or more of these. Examples of the pharmaceutically acceptable salt of rotigotine include acid addition salts, and examples of the acid of the acid addition salt include hydrochloric acid, sulfuric acid, nitric acid, acetic acid, phosphoric acid, phosphorous acid, hydrobromic acid, maleic acid, malic acid, ascorbic acid, tartaric acid, lauric acid, stearic acid, palmitic acid, oleic acid, myristic acid, lauryl sulfuric acid, linolenic acid, and fumaric acid. The acid addition salt may be one or two or more of these acid addition salts. Among these, the adhesive agent layer according to the present invention preferably comprises rotigotine in a free form.

In the present invention, the content of rotigotine and/or a pharmaceutically acceptable salt thereof comprised in the adhesive agent layer (the content of rotigotine or the content of a pharmaceutically acceptable salt of rotigotine, or the total content thereof when both are comprised, the same shall apply hereinafter) is preferably 5 to 15% by mass relative to the total mass of the adhesive agent layer in terms of rotigotine free form, more preferably 6 to 12% by mass, still more preferably 7 to 10% by mass, and even more preferably 8 to 9% by mass. If the content of rotigotine and/or a pharmaceutically acceptable salt thereof is less than the lower limit, the skin permeability of rotigotine and/or a pharmaceutically acceptable salt thereof tends to decrease, whereas if it exceeds the upper limit, the crystals of rotigotine tend to precipitate, or the adhesive force of the adhesive agent layer tends to decrease.

According to the rotigotine-containing patch of the present invention and the method for improving rotigotine stability of the present invention described below, it is possible to sufficiently suppress the generation of degradation products of rotigotine and/or a pharmaceutically acceptable salt thereof (rotigotine degradation products). Examples of such degradation products include, but are not limited to, 7,8-dihydronaphthol and despropyl rotigotine (Despropyl RTN).

### <Adhesive Base Agent>

The adhesive agent layer according to the present invention further comprises an adhesive base agent. The adhesive base agent is not particularly limited, and examples thereof include rubber-based adhesive base agents, acrylic-based adhesive base agents, and silicone-based adhesive base agents, and may be one or a combination of two or more of these, but is preferably at least one selected from the group consisting of a rubber-based adhesive base agent, a carboxy group-free acrylic-based adhesive base agent, and a silicone-based adhesive base agent, and more preferably comprises at least a rubber-based adhesive base agent.

In the adhesive agent layer according to the present invention, the content of the adhesive base agent (the total content thereof when two or more are used, the same shall apply hereinafter) is preferably 1 to 90% by mass, more preferably 5 to 90% by mass, still more preferably 10 to 90% by mass, and even more preferably 10 to 80% by mass relative to the total mass of the adhesive agent layer.

### (Rubber-Based Adhesive Base Agent)

Examples of the rubber-based adhesive base agent include styrene-based thermoplastic elastomers, polyisobutylene, natural rubber, alkyl vinyl ether (co)polymers, polyisoprene, and polybutadiene, and one of these may be used alone, or two or more of these may be used in combination, but among these, styrene-based thermoplastic elastomers are particularly preferable. The styrene-based thermoplastic elastomer is a styrene-based elastomer that exhibits thermoplasticity in that it softens and exhibits fluidity when heated and returns to a rubber-like elastic body when cooled. Among these, styrene-based block copolymers are preferable from the viewpoints of sufficient adhesiveness and excellent stability over time of rotigotine and/or a pharmaceutically acceptable salt thereof.

Specific examples of the styrene-based block copolymer include styrene-butadiene block copolymers, styrene-butadiene-styrene block copolymers, styrene-isoprene block copolymers, styrene-isoprene-styrene block copolymers, styrene-ethylene/butylene block copolymers, styrene-ethylene/butylene-styrene block copolymers, styrene-ethylene/propylene block copolymers, styrene-ethylene/propylene-styrene block copolymers, styrene-isobutylene block copolymers, and styrene-isobutylene-styrene block copolymers, and one of these may be used alone, or two or more of these may be used in combination. In the above, "ethylene/butylene" represents a copolymer block of ethylene and butylene, and "ethylene/propylene" represents a copolymer block of ethylene and propylene. Among these, the styrene-based thermoplastic elastomer according to the present invention is more preferably a styrene-isoprene-styrene block copolymer.

The styrene-isoprene-styrene block copolymer preferably has a viscosity-average molecular weight of 30,000 to 2,500,000, more preferably 100,000 to 1,700,000. If the viscosity-average molecular weight is less than the lower limit, the preparation physical properties (particularly, the cohesive force of the adhesive agent layer) of the patch tend to decrease, whereas if it exceeds the upper limit, compatibility with other components comprised in the adhesive agent layer tends to decrease, making it difficult to produce the patch.

In the present invention, when the styrene-based thermoplastic elastomer is comprised as the adhesive base agent in the adhesive agent layer, the content thereof (the total content thereof when the styrene-based thermoplastic elastomer is a combination of two or more, the same shall apply hereinafter) is preferably 5 to 50% by mass, more preferably 10 to 40% by mass, and still more preferably 10 to 30% by mass relative to the total mass of the adhesive agent layer. If the content of the styrene-based thermoplastic elastomer is less than the lower limit, the cohesive force, shape retention, and the like of the adhesive agent layer tend to decrease, whereas if it exceeds the upper limit, the cohesive force of the adhesive agent layer excessively increases, so that the adhesive force of the adhesive agent layer tends to decrease, or compatibility tends to decrease.

Further, the rubber-based adhesive base agent is more preferably a combination of the styrene-based thermoplastic elastomer (more preferably, the styrene-isoprene-styrene block copolymer) and the polyisobutylene, and the mass ratio of the styrene-based thermoplastic elastomer to the polyisobutylene (mass of the styrene-based thermoplastic elastomer:mass of the polyisobutylene) is more preferably 1:2 to 30:1 (still more preferably 1:1 to 10:1), from the viewpoint that the adhesive force and cohesive force of the adhesive agent layer tend to be further improved.

Specific examples of the styrene-isoprene-styrene block copolymer include QUINTAC (registered trademark) 3570C (trade name, manufactured by ZEON CORPORATION), SIS5002, SIS5229, SIS5505, and SIS5505P (trade name, manufactured by JSR Corporation), SIBSTAR (registered trademark) T102 (trade name, manufactured by Kaneka Corporation), and the like. Polyisobutylene also includes so-called butyl rubber (isobutylene-isoprene rubber), and specific examples thereof include Oppanol (registered trademark) N50, N80, N100, N150, B11, B12, B50, B80, B100, B120, B150, and B220 (trade name, manufactured by BASF), JSR (registered trademark) Butyl065, 268, and 365 (trade name, manufactured by JSR Corporation), X_Butyl (registered trademark) RB 100, 101-3, 301, and 402 (trade name, manufactured by ARLANXEO), Exxon (registered trademark) Butyl065, 065S, 068, 068S, 268, 268S, 365, and 365S (trade name, manufactured by Exxon Mobile), Butyl065, 268, and 365 (trade name, manufactured by Japan Butyl Co., Ltd.), and the like.

In the present invention, when the rubber-based adhesive base agent is comprised as the adhesive base agent in the adhesive agent layer, the content thereof (the total content thereof when two or more are used in combination, the same shall apply hereinafter) is preferably 1 to 60% by mass, more preferably 5 to 50% by mass, and still more preferably 10 to 40% by mass relative to the total mass of the adhesive agent layer.

### (Acrylic-Based Adhesive Base Agent)

Examples of the acrylic-based adhesive base agent according to the present invention include those listed as adhesive agents in "Japanese Pharmaceutical Excipients Directory 2016 (edited by International Pharmaceutical Excipients Council Japan)," and one or a combination of two or more of these may be used, but a carboxy group-free acrylic-based adhesive base agent is preferable, and a functional group-free acrylic-based adhesive base agent is more preferable. In the present invention, the "carboxy group-free acrylic-based adhesive base agent" and the "functional group-free acrylic-based adhesive base agent" respectively refer to acrylic polymers substantially free of carboxy groups and functional groups, preferably, having a content of carboxy groups and functional groups in the polymer of less than 3% by mass, respectively.

Examples of the carboxy group-free acrylic-based adhesive base agent include substantially functional group-free acrylic-based adhesive base agents such as 2-ethylhexyl acrylate-vinylpyrrolidone copolymer, 2-ethylhexyl acrylate-2-ethylhexyl methacrylate-dodecyl methacrylate copolymer, 2-ethylhexyl acrylate-vinyl acetate copolymer, 2-ethylhexyl acrylate-methyl methacrylate-butyl acrylate copolymer, and ethyl acrylate-methyl methacrylate copolymer; and acrylic-based adhesive base agents having a hydroxy group such as 2-ethylhexyl (meth)acrylate-vinyl acetate-2-hydroxyethyl acrylate copolymer, 2-hydroxyethyl (meth)acrylate copolymer, 2-hydroxypropyl (meth)acrylate copolymer, 3-hydroxypropyl (meth)acrylate copolymer, 4-hydroxybutyl (meth)acrylate copolymer, and 2-ethylhexyl acrylate-vinyl acetate-hydroxyethyl acrylate-glycidyl methacrylate copolymer, and one of these may be used alone, or two or more of these may be used in combination.

As the carboxy group-free acrylic-based adhesive base agent, a commercially available product may be appropriately used, and examples thereof include acrylic polymers contained in MAS 811 and MAS 683 (manufactured by CosMED Pharmaceutical Co., Ltd.); Duro-Tak (registered trademark) acrylic adhesive agent series (manufactured by Henkel) 87-900A, 87-901A, 87-9301, 87-4098, 87-9088, and 87-9085; and GELVA (registered trademark) acrylic adhesive agent series (manufactured by Henkel) GMS 3083, GMS 3253, and GMS3235, and acrylic polymers contained in Duro-Tak (registered trademark) acrylic adhesive agent series (manufactured by Henkel) 87-202A, 87-2287, 87-2516, 87-2510, 87-4287, 87-2525, 87-201A, 87-202A, 87-208A, 87-502A, 87-503A, and 87-504A; and GELVA (registered trademark) acrylic adhesive agent series (manufactured by Henkel) GMS 788 and GMS 737, and the like can be appropriately used.

When the adhesive agent layer according to the present invention comprises the acrylic-based adhesive base agent, the content thereof (the total content thereof when two or more are used, the same shall apply hereinafter) is preferably 10 to 90% by mass and more preferably 20 to 80% by mass relative to the total mass of the adhesive agent layer. If the content of the acrylic-based adhesive base agent is less than the lower limit, the cohesive force of the adhesive agent layer tends to decrease, whereas if it exceeds the upper limit, the skin permeability of rotigotine and/or a pharmaceutically acceptable salt thereof tends to decrease.

### (Silicone-Based Adhesive Base Agent)

The silicone-based adhesive base agent according to the present invention includes silicone rubbers represented by MQ (polydimethylsiloxane), VMQ (polymethylvinylsiloxane), PMQ (polymethylphenylsiloxane), and PVMQ (polyphenylvinylmethylsiloxane) in ASTM standards (ASTM D 1418), and mixtures of at least one of these and a silicone resin other than silicone rubber, such as polyditrimethylsilylsiloxane, and one of these may be used alone, or two or more of these may be used in combination. In addition, when the silicone resin other than the silicone rubber is mixed, it is preferably 0.1 to 20% by mass relative to the total mass of the silicone-based adhesive base agent.

Further, as these silicone-based adhesive base agents, commercially available ones may be appropriately used, and for example, silicone-based adhesive base agents provided by DuPont-Toray Specialty Materials K.K. under the following model numbers: BIO-PSA7-410X, BIO-PSA7-420X, BIO-PSA7-430X, BIO-PSA7-440X, BIO-PSA7-450X, BIO-PSA7-460X (X is each independently 1 or 2), BIO-PSA AC7-4201, BIO-PSA AC7-4301, BIO-PSA AC7-4302, MD7-4502, MD7-4602, 7-9700, MG7-9800, MG7-9850; and BIO-PSA 7-4560 (hot melt silicone adhesive agent), and the like can be appropriately used.

Furthermore, examples of the silicone-based adhesive base agent according to the present invention include: when having methyl groups, one obtained by dehydrogenating hydrogen atoms of the methyl groups and crosslinking the methyl groups by adding a peroxide; when having vinyl groups, one obtained by binding a crosslinking agent composed of a SiH group-containing siloxane compound to crosslink the vinyl groups; and when having hydroxyl groups (that is, when having silanol groups), one obtained by crosslinking the silanol groups by dehydration condensation.

When the adhesive agent layer according to the present invention comprises the silicone-based adhesive base agent, the content thereof (the total content thereof when two or more are used, the same shall apply hereinafter) is preferably 10 to 90% by mass and more preferably 20 to 80% by mass relative to the total mass of the adhesive agent layer. If the content of the silicone-based adhesive base agent is less than the lower limit, the cohesive force of the adhesive agent layer tends to decrease, whereas if it exceeds the upper limit, the skin permeability of rotigotine and/or a pharmaceutically acceptable salt thereof tends to decrease.

### <Propyl Gallate>

In the rotigotine-containing patch of the present invention, the adhesive agent layer comprises propyl gallate. The content of propyl gallate comprised in the adhesive agent layer according to the present invention is preferably 0.01 to 1% by mass, more preferably 0.05 to 1% by mass, still more preferably 0.075 to 0.8% by mass, even more preferably 0.1 to 0.6% by mass, and particularly preferably 0.1 to 0.3% by mass relative to the total mass of the adhesive agent layer. If the content of propyl gallate is less than the lower limit, the effect of improving the stability over time of rotigotine and/or a pharmaceutically acceptable salt thereof tends not to be sufficiently exhibited, whereas if it exceeds the upper limit, the cohesive force of the adhesive agent layer tends to decrease, or the adhesive force tends to decrease.

Further, the content of propyl gallate comprised in the adhesive agent layer is preferably 0.001 to 0.12 parts by mass, more preferably 0.005 to 0.115 parts by mass, still more preferably 0.008 to 0.09 parts by mass, even more preferably 0.01 to 0.07 parts by mass, and particularly preferably 0.01 to 0.035 parts by mass relative to 1 part by mass of the content of rotigotine and/or a pharmaceutically acceptable salt thereof in terms of rotigotine free form. When the ratio of the content of propyl gallate to the content of rotigotine and/or a pharmaceutically acceptable salt thereof is within the above range, the stability over time of rotigotine and/or a pharmaceutically acceptable salt thereof tends to be particularly exhibited.

### <Thioglycolic Acid and Pharmaceutically Acceptable Salt Thereof>

The adhesive agent layer according to the present invention may further comprise at least one selected from the group consisting of thioglycolic acid and a pharmaceutically acceptable salt of thioglycolic acid (thioglycolic acid and/or a pharmaceutically acceptable salt thereof) from the viewpoint of improving the stability over time of rotigotine and/or a pharmaceutically acceptable salt thereof.

In the present invention, the form of thioglycolic acid which may be comprised in the adhesive agent layer may be a free form or a pharmaceutically acceptable salt thereof, and a pharmaceutically acceptable salt of thioglycolic acid may be desalted during production and/or in the produced preparation to be in a free form, and may be one or a mixture of two or more of these. Examples of the pharmaceutically acceptable salt of thioglycolic acid include alkali metal salts, alkaline earth metal salts, salts with ammonia, alkylamines, and alkanolamines, and more specifically, sodium salt, potassium salt, calcium salt, magnesium salt, ammonium salt, dimethylamine salt, diethylamine salt, trimethylamine salt, triethylamine salt, monoethanolamine salt, diethanolamine salt, diisopropanolamine salt, triethanolamine salt, and triisopropanolamine salt. The pharmaceutically acceptable salt of thioglycolic acid may be one or two or more of these salts. The form of thioglycolic acid which may be comprised in the adhesive agent layer according to the present invention is preferably at least one selected from the group consisting of thioglycolic acid and an alkali metal salt (more preferably sodium salt) of thioglycolic acid.

When the adhesive agent layer according to the present invention further comprises thioglycolic acid and/or a pharmaceutically acceptable salt thereof, the content of thioglycolic acid and/or a pharmaceutically acceptable salt thereof comprised in the adhesive agent layer (the content of thioglycolic acid or the content of a pharmaceutically acceptable salt of thioglycolic acid, or the total content thereof when both are comprised, the same shall apply hereinafter) is preferably 0.03 to 3% by mass relative to the total mass of the adhesive agent layer in terms of sodium thioglycolate, more preferably 0.05 to 3% by mass, still more preferably 0.1 to 3% by mass, even more preferably 0.15 to 3% by mass, and particularly preferably 0.25 to 3% by mass or 0.5 to 3% by mass. If the content of thioglycolic acid and/or a pharmaceutically acceptable salt thereof is less than the lower limit, the effect of improving the stability over time of rotigotine and/or a pharmaceutically acceptable salt thereof by thioglycolic acid and/or a pharmaceutically acceptable salt thereof tends not to be sufficiently exhibited, whereas if it exceeds the upper limit, the adhesive force of the adhesive agent layer tends to decrease.

Further, the content of thioglycolic acid and/or a pharmaceutically acceptable salt thereof comprised in the adhesive agent layer in terms of sodium thioglycolate is preferably 0.02 to 300 parts by mass, more preferably 0.04 to 60 parts by mass, still more preferably 0.1 to 40 parts by mass, even more preferably 0.2 to 30 parts by mass, and particularly preferably 0.8 to 30 parts by mass relative to 1 part by mass of the content of propyl gallate in the adhesive agent layer. When the content of thioglycolic acid and/or a pharmaceutically acceptable salt thereof relative to the content of propyl gallate is within the above range, the effect of improving the stability over time of rotigotine and/or a pharmaceutically acceptable salt thereof by thioglycolic acid and/or a pharmaceutically acceptable salt thereof tends to be particularly exhibited.

Furthermore, the content of thioglycolic acid and/or a pharmaceutically acceptable salt thereof comprised in the adhesive agent layer in terms of sodium thioglycolate is preferably 0.002 to 0.6 parts by mass, more preferably 0.01 to 0.6 parts by mass, still more preferably 0.015 to 0.6 parts by mass, even more preferably 0.03 to 0.35 parts by mass, and particularly preferably 0.05 to 0.35 parts by mass relative to 1 part by mass of the content of rotigotine and/or a pharmaceutically acceptable salt thereof in terms of rotigotine free form. When the ratio of the content of thioglycolic acid and/or a pharmaceutically acceptable salt thereof to the content of rotigotine and/or a pharmaceutically acceptable salt thereof is within the above range, the effect of improving the stability over time of rotigotine and/or a pharmaceutically acceptable salt thereof by thioglycolic acid and/or a pharmaceutically acceptable salt thereof tends to be particularly exhibited.

However, the adhesive agent layer according to the present invention is also preferably substantially free of both thioglycolic acid and a pharmaceutically acceptable salt thereof, from the viewpoint of suppressing unpleasant odors during the production of the patch. Being substantially free of thioglycolic acid and a pharmaceutically acceptable salt thereof means that the content of thioglycolic acid and a pharmaceutically acceptable salt thereof in terms of sodium thioglycolate is less than 0.03% by mass (for example, 0.029% by mass or less) relative to the total mass of the adhesive agent layer.

### <Other Components>

The adhesive agent layer according to the present invention may further comprise other drugs than rotigotine and a pharmaceutically acceptable salt thereof; other stabilizers than propyl gallate and thioglycolic acid and a pharmaceutically acceptable salt thereof; tackifiers; absorption enhancers; dermal anti-irritants; and additives such as absorbents, desalting agents, plasticizers, solubilizers, fillers, and preservatives, within a range not impairing the effects of the present invention.

### (Other Drugs)

Examples of the other drugs than rotigotine and a pharmaceutically acceptable salt thereof include non-steroidal anti-inflammatory analgesics (diclofenac, indomethacin, ketoprofen, felbinac, loxoprofen, ibuprofen, flurbiprofen, tiaprofenic acid, acemetacin, sulindac, etodolac, tolmetin, piroxicam, meloxicam, ampiroxicam, naproxen, azapropazone, methyl salicylate, glycol salicylate, valdecoxib, celecoxib, rofecoxib, amfenac, etc.), antipyretic analgesics (acetaminophen, etc.), antihistamines (diphenhydramine, chlorpheniramine, mequitazine, homochlorcyclizine, etc.), antihypertensives (diltiazem, nicardipine, nilvadipine, metoprolol, bisoprolol, trandolapril, etc.), antiparkinsonian agents (pergolide, ropinirole, bromocriptine, selegiline, etc.), bronchodilators (tulobuterol, isoproterenol, salbutamol, etc.), antiallergic agents (ketotifen, loratadine, azelastine, terfenadine, cetirizine, acitazanolast, etc.), local anesthetics (lidocaine, dibucaine, etc.), therapeutic agents for neuropathic pain (pregabalin, etc.), non-narcotic analgesics (buprenorphine, tramadol, pentazocine, etc.), narcotic analgesics (morphine, oxycodone, fentanyl, etc.), urological agents (oxybutynin, tamsulosin, etc.), psychoneurological agents (promazine, chlorpromazine, etc.), steroid hormones (estradiol, progesterone, norethisterone, cortisone, hydrocortisone, etc.), antidepressants (sertraline, fluoxetine, paroxetine, citalopram, etc.), antidementia drugs (donepezil, rivastigmine, galantamine, etc.), antipsychotics (risperidone, olanzapine, etc.), central nervous system stimulants (methylphenidate, etc.), therapeutic agents for osteoporosis (raloxifene, alendronate, etc.), prophylactic agents for breast cancer (tamoxifen, etc.), antiobesity drugs (mazindol, sibutramine, etc.), therapeutic agents for insomnia (melatonin, etc.), and antirheumatic drugs (actarit, etc.), and one or a combination of two or more of these may be used.

When the adhesive agent layer according to the present invention further comprises the other drugs, the content thereof (the total content thereof when two or more are used) is preferably 10% by mass or less relative to the total mass of the adhesive agent layer.

### (Other Stabilizers)

Examples of other stabilizers than propyl gallate and thioglycolic acid and a pharmaceutically acceptable salt thereof include ascorbic acid or a metal salt or ester thereof (for example, palmitic acid ester), isoascorbic acid or a metal salt thereof, ethylenediaminetetraacetic acid or a metal salt thereof, cysteine, acetylcysteine, 2-mercaptobenzimidazole, dibutylhydroxytoluene, butylhydroxyanisole, pentaerythrityl tetrakis[3-(3,5-di-t-butyl-4-hydroxyphenyl)propionate], 3-mercapto-1,2-propanediol, tocopherol, tocopherol acetate, thymol, soy lecithin, rutin, dihydroxybenzoic acid, potassium dichloroisocyanurate, quercetin, hydroquinone, hydroxymethanesulfinic acid metal salt, meta-bisulfite metal salt (for example, sodium metabisulfite), sulfite metal salt, and thiosulfate metal salt other than thioglycolic acid metal salt, and one or a combination of two or more of these may be used. In the above, examples of the metal salt include sodium salt, potassium salt, calcium salt, magnesium salt, and calcium disodium salt. Further, examples of the ester include palmitic acid ester, stearic acid ester, and myristic acid ester.

However, in the adhesive agent layer according to the present invention, when the effect of stabilizing the stability over time of rotigotine and/or a pharmaceutically acceptable salt thereof by propyl gallate is inhibited, or from the viewpoint of the adhesive force of the adhesive agent layer, it is preferable that the adhesive agent layer does not comprise the other stabilizers, and the content thereof (the total content thereof when two or more are used) is preferably 3% by mass or less, more preferably 2% by mass or less, and still more preferably 0.25% by mass or less (for example, 0 to 0.25% by mass, 0.005 to 0.25% by mass) relative to the total mass of the adhesive agent layer.

### (Tackifier)

The tackifier is blended mainly for the purpose of enhancing the adhesive properties of the adhesive base agent (preferably, the rubber-based adhesive base agent). Examples of such a tackifier include petroleum resins, rosin-based resins, terpene-based resins, phenol-based resins, and xylene-based resins. The tackifier may be one or a combination of two or more of these, but when the adhesive agent layer according to the present invention comprises the rubber-based adhesive base agent as the adhesive base agent, it is preferable that the petroleum resin is further comprised.

When the tackifier is further comprised in the adhesive agent layer, the content thereof (the total content thereof when two or more are used) is preferably 5 to 80% by mass, more preferably 10 to 80% by mass, and still more preferably 20 to 60% by mass relative to the total mass of the adhesive agent layer, from the viewpoint of improving the adhesive force of the adhesive agent layer and/or reducing local irritation during peeling.

Examples of the petroleum resin include C5-based synthetic petroleum resins (copolymers of at least two of isoprene, cyclopentadiene, 1,3-pentadiene, and 1-pentene; copolymers of at least two of 2-pentene and dicyclopentadiene; resins mainly composed of 1,3-pentadiene, etc.), C9-based synthetic petroleum resins (copolymers of at least two of indene, styrene, methylindene, and α-methylstyrene, etc.), and dicyclopentadiene-based synthetic petroleum resins (copolymers mainly composed of dicyclopentadiene with isoprene and/or 1,3-pentadiene). Further, from the viewpoint of another classification, for example, alicyclic petroleum resins (such as alicyclic saturated hydrocarbon resins), alicyclic hydrogenated petroleum resins, aliphatic petroleum resins (such as aliphatic hydrocarbon resins), aliphatic hydrogenated petroleum resins, and aromatic petroleum resins, and more specifically, ARKON P-70, ARKON P-85, ARKON P-90, ARKON P-100, ARKON P-115, ARKON P-125, ARKON M-90, ARKON M-100, ARKON M-115, ARKON M-135 (all the foregoing are trade names and manufactured by Arakawa Chemical Industries, Ltd.), and ESCOREZ 8000 (trade name, manufactured by ESSO SEKIYU KAGAKU K.K.) are included. The petroleum resin according to the present invention may be one or a combination of two or more of these. Among these, alicyclic saturated hydrocarbon resins are more preferable from the viewpoints that suitable adhesion to the skin is easily obtained, there is little odor and the like, so that the feeling in use is good, and the generation of rotigotine degradation products is further suppressed.

In the present invention, the alicyclic saturated hydrocarbon resin refers to a resin which is a homopolymer or copolymer of an alicyclic saturated hydrocarbon monomer. The alicyclic saturated hydrocarbon resin preferably has a weight-average molecular weight of 1,000 to 2,300, more preferably 1,000 to 1,800, still more preferably 1,000 to 1,600, even more preferably 1,000 to 1,500, and particularly preferably 1,200 to 1,400.

When the adhesive agent layer according to the present invention further comprises the petroleum resin (preferably, the alicyclic saturated hydrocarbon resin), the content thereof (the total content thereof when two or more are used) is preferably 5 to 80% by mass, more preferably 10 to 70% by mass, still more preferably 10 to 60% by mass, and particularly preferably 20 to 60% by mass relative to the total mass of the adhesive agent layer. If the content of the petroleum resin is less than the lower limit, the adhesive force of the adhesive agent layer and the adhesion to the skin tend to decrease, whereas if it exceeds the upper limit, the skin permeability of rotigotine and/or a pharmaceutically acceptable salt thereof and the shape retention of the adhesive agent layer tend to decrease.

### (Absorption Enhancer)

Examples of the absorption enhancer include those having a transdermal absorption promoting action (skin permeation promoting action) of drugs, and for example, aliphatic alcohols, fatty acids having 6 to 20 carbon atoms, fatty acid esters, fatty acid amides, or fatty alcohol ethers; aromatic organic acids; aromatic alcohols; aromatic organic acid esters or ethers; POE-hydrogenated castor oils; lecithins; phospholipids; soybean oil derivatives; and triacetin. The absorption enhancer may be one or a combination of two or more of these, but from the viewpoint of superior skin permeability of rotigotine and/or a pharmaceutically acceptable salt thereof, it is preferable that an aliphatic alcohol is further comprised in the adhesive agent layer.

When the absorption enhancer is further comprised in the adhesive agent layer, the content thereof (the total content thereof when two or more are used) is preferably 1 to 15% by mass, more preferably 3 to 7% by mass relative to the total mass of the adhesive agent layer, from the viewpoint of superior skin permeability of rotigotine and/or a pharmaceutically acceptable salt thereof.

The aliphatic alcohol refers to a saturated or unsaturated, linear or branched, monohydric or polyhydric aliphatic alcohol, and the aliphatic alcohol according to the present invention is preferably monohydric. The aliphatic alcohol preferably has 3 to 23 carbon atoms, more preferably 12 to 23 carbon atoms, and still more preferably 12 to 20 carbon atoms. If the number of carbon atoms of the aliphatic alcohol is less than the lower limit, the boiling point becomes low, so that it becomes difficult to keep the content in the preparation constant, and the stability over time of the aliphatic alcohol tends to decrease, whereas if it exceeds the upper limit, the skin permeability of rotigotine and/or a pharmaceutically acceptable salt thereof tends to decrease.

Examples of the aliphatic alcohol include isopropanol, hexyl alcohol, lauryl alcohol, myristyl alcohol, cetyl alcohol, stearyl alcohol, isostearyl alcohol, octyldodecanol, oleyl alcohol, linolenyl alcohol, and hexyldecanol. The aliphatic alcohol may be one or a combination of two or more of these, but from the viewpoint of further improving the skin permeability of rotigotine and/or a pharmaceutically acceptable salt thereof, in addition to the viewpoints of the stability over time and compatibility of the above aliphatic alcohols, at least one selected from the group consisting of octyldodecanol and lauryl alcohol is preferable.

When the adhesive agent layer according to the present invention further comprises the aliphatic alcohol, the content thereof (the total content thereof when two or more are used) is preferably 1 to 15% by mass, more preferably 1 to 10% by mass, still more preferably 2 to 7% by mass, and particularly preferably 3 to 7% by mass relative to the total mass of the adhesive agent layer. If the content of the aliphatic alcohol is less than the lower limit, the skin permeability of rotigotine and/or a pharmaceutically acceptable salt thereof tends not to be sufficiently improved, whereas if it exceeds the upper limit, the compatibility with the adhesive base agent and other components tends to decrease.

### (Dermal Anti-Irritant)

Examples of the dermal anti-irritant include those having an action of reducing irritation to the skin caused by drugs and stabilizers, and for example, cholesterol is included. The dermal anti-irritant may be a combination of two or more.

When the dermal anti-irritant is further comprised in the adhesive agent layer, the content thereof (the total content thereof when two or more are used) is preferably 0.1 to 7% by mass, more preferably 1 to 7% by mass, and still more preferably 3 to 5% by mass relative to the total mass of the adhesive agent layer, from the viewpoint that a sufficient skin irritation reducing effect is produced and compatibility with an adhesive base agent is sufficiently maintained.

### (Additives)

### [Absorbent]

Examples of the absorbent include inorganic and/or organic substances having hygroscopicity, and more specifically, minerals such as talc, kaolin, and bentonite; silicon compounds such as fumed silica (such as AEROSIL (registered trademark)) and hydrous silica; metal compounds such as zinc oxide and dried aluminum hydroxide gel; weak acids such as lactic acid and acetic acid; sugars such as dextrin; and polymers such as polyvinylpyrrolidone (non-crosslinked PVP), crosslinked polyvinylpyrrolidone (also referred to as "crospovidone" or "crosslinked PVP"), aminoalkyl methacrylate copolymer, carboxyvinyl polymer, and butyl methacrylate methyl methacrylate copolymer. The absorbent may be one or a combination of two or more of these, but from the viewpoint of superior stability over time of rotigotine and/or a pharmaceutically acceptable salt thereof, it is preferable that crosslinked polyvinylpyrrolidone is further comprised in the adhesive agent layer.

When the absorbent is further comprised in the adhesive agent layer, the content thereof (the total content thereof when two or more are used) is preferably 3 to 25% by mass and more preferably 3 to 15% by mass relative to the total mass of the adhesive agent layer, from the viewpoint of the adhesive properties of the adhesive agent layer.

Examples of the crosslinked polyvinylpyrrolidone include crosslinked N-vinylpyrrolidone polymers. The N-vinylpyrrolidone polymer may be a homopolymer or a copolymer, and examples thereof include a homopolymer of N-vinylpyrrolidone and a copolymer of N-vinylpyrrolidone and a polyfunctional monomer. Among these, the crosslinked polyvinylpyrrolidone according to the present invention is preferably a crosslinked homopolymer of 1-vinyl-2-pyrrolidone (also referred to as "crospovidone"). As crospovidone, commercially available products such as Kollidon CL and Kollidon CL-M (manufactured by BASF Japan Ltd.); and Polyplasdone XL, Polyplasdone XL-10, and Polyplasdone INF-10 (manufactured by ISP Japan K.K.) may be used.

When the adhesive agent layer according to the present invention further comprises the crosslinked polyvinylpyrrolidone, the content thereof (the total content thereof when two or more are used) is preferably 3 to 25% by mass, more preferably 3 to 20% by mass, and still more preferably 3 to 15% by mass relative to the total mass of the adhesive agent layer. If the content of the crosslinked polyvinylpyrrolidone is less than the lower limit, the crystals of rotigotine and/or a pharmaceutically acceptable salt thereof tend to easily precipitate, whereas if it exceeds the upper limit, the skin permeability of rotigotine and/or a pharmaceutically acceptable salt thereof tends to decrease, or the compatibility in the adhesive agent layer composition tends to decrease during production, making it difficult to perform production.

Further, the content of crosslinked polyvinylpyrrolidone comprised in the adhesive agent layer is preferably 15:3 to 5:25, more preferably 15:3 to 5:20, and still more preferably 15:3 to 5:15 in terms of the mass ratio of the content of rotigotine and/or a pharmaceutically acceptable salt thereof comprised in the adhesive agent layer in terms of rotigotine free form (content of rotigotine and/or a pharmaceutically acceptable salt thereof in terms of rotigotine free form:content of crosslinked polyvinylpyrrolidone). If the ratio of the content of crosslinked polyvinylpyrrolidone to the content of rotigotine and/or a pharmaceutically acceptable salt thereof is less than the lower limit, the crystals of rotigotine tend to precipitate, whereas if it exceeds the upper limit, the skin permeability of rotigotine and/or a pharmaceutically acceptable salt thereof tends to decrease.

### [Desalting Agent]

The desalting agent is blended mainly for the purpose of converting all or part of a basic drug into a free form. The desalting agent is not particularly limited, but for example, when a pharmaceutical preparation comprising a free drug is obtained by blending an acid addition salt of a drug as the drug, a basic substance is preferable, and a metal ion-containing desalting agent and a basic nitrogen atom-containing desalting agent are more preferable. Examples of the metal ion-containing desalting agent include sodium acetate (including anhydrous sodium acetate), sodium hydroxide, potassium hydroxide, magnesium hydroxide, calcium hydroxide, sodium hydrogen carbonate, potassium hydrogen carbonate, sodium citrate, and sodium lactate, and one or a combination of two or more of these may be used. The adhesive agent layer according to the present invention may further comprise a compound derived from the basic drug and the desalting agent (for example, sodium chloride produced when rotigotine hydrochloride and sodium acetate are combined). In the present invention, when these desalting agents and a compound derived from a basic drug and a desalting agent are further comprised in the adhesive agent layer, the content thereof is preferably 10% by mass or less in total when two or more are used relative to the total mass of the adhesive agent layer.

### [Plasticizer]

The plasticizer is blended mainly for the purpose of adjusting the adhesive properties of the adhesive agent layer, the flow properties in the production of the adhesive agent layer, the transdermal absorption properties of the drug, and the like. Examples of such a plasticizer include silicone oils; petroleum-based oils such as paraffinic process oils, naphthenic process oils, and aromatic process oils; squalane and squalene; vegetable-based oils such as olive oil, camellia oil, castor oil, tall oil, and peanut oil; dibasic acid esters such as dibutyl phthalate and dioctyl phthalate; liquid rubbers such as liquid polybutene and liquid isoprene rubber; and diethylene glycol, polyethylene glycol, propylene glycol, and dipropylene glycol. The plasticizer may be one or a combination of two or more of these, but is particularly preferably at least one selected from the group consisting of silicone oil, liquid paraffin, and liquid polybutene.

When the adhesive agent layer according to the present invention further comprises the plasticizer, the content thereof (the total content thereof when two or more are used) is preferably 1 to 30% by mass, more preferably 5 to 20% by mass relative to the total mass of the adhesive agent layer, from the viewpoint of improving the adhesive force of the adhesive agent layer and/or reducing local irritation during peeling.

### [Solubilizer, Filler]

Examples of the solubilizer include organic acids such as acetic acid and surfactants, and one or a combination of two or more of these may be used. The filler is blended mainly for the purpose of adjusting the adhesive force of the adhesive agent layer, and examples thereof include aluminum hydroxide, calcium carbonate, and magnesium carbonate; silicates such as aluminum silicate and magnesium silicate; silicic acid, barium sulfate, calcium sulfate, calcium zincate, zinc oxide, and titanium oxide, and one or a combination of two or more of these may be used.

### [Preservative]

Examples of the preservative include p-hydroxybenzoic acid derivatives, benzyl alcohol, phenol, and cresol, and one or a combination of two or more of these may be used.

The adhesive agent layer according to the present invention is not particularly limited, but the mass per unit area (area of adhesive surface) is preferably 20 to 200 g/m², more preferably 30 to 100 g/m², and still more preferably 30 to 70 g/m². The area of the adhesive surface of the adhesive agent layer according to the present invention can be appropriately adjusted according to the purpose of treatment and the subject of application, and is not particularly limited, but is usually in the range of 0.5 to 200 cm².

### [Method for Improving Rotigotine Stability, Method for Producing Rotigotine-Containing Patch]

The method for improving rotigotine stability of the present invention is a method for improving stability of at least one selected from the group consisting of rotigotine and a pharmaceutically acceptable salt of rotigotine in a rotigotine-containing patch including a backing layer and an adhesive agent layer, wherein the adhesive agent layer comprises an adhesive base agent and at least one selected from the group consisting of rotigotine and a pharmaceutically acceptable salt of rotigotine, the method including a step of further comprising propyl gallate in the adhesive agent layer. Further, since the above-described rotigotine-containing patch of the present invention can be obtained by such a method for improving rotigotine stability, this can also be regarded as a method for producing a rotigotine-containing patch (hereinafter, the method for improving rotigotine stability and the method for producing a rotigotine-containing patch are collectively referred to simply as "the method of the present invention" in some cases).

In the method of the present invention, the method of further comprising propyl gallate in the adhesive agent layer is not particularly limited, and a known method for producing a patch can be appropriately adopted. For example, first, propyl gallate is added to rotigotine and/or a pharmaceutically acceptable salt thereof, and the adhesive base agent, and, if necessary, thioglycolic acid and/or a pharmaceutically acceptable salt thereof, the other components described above, and an appropriate amount of a solvent, and the mixture is kneaded according to a conventional method to obtain a uniform adhesive agent layer composition. When rotigotine free form is used as rotigotine and/or a pharmaceutically acceptable salt thereof, type I crystals, type II crystals, or an amorphous form thereof may be used, or a mixture of at least two of type I crystals, type II crystals, and an amorphous form may be used. Further, rotigotine and/or a pharmaceutically acceptable salt thereof may be a hydrate, or a solution thereof in the solvent may be used. Examples of the solvent include absolute ethanol, toluene, heptane, methanol, ethyl acetate, hexane, isopropanol, and a mixed solution of at least two of these.

The order of blending of the respective components at this time, that is, rotigotine and/or a pharmaceutically acceptable salt thereof, the adhesive base agent, propyl gallate, and, if necessary, thioglycolic acid and/or a pharmaceutically acceptable salt thereof and the other components, is not particularly limited, and the blending amounts thereof are preferably amounts such that the content of each component in the resulting adhesive agent layer becomes the respective content described in the rotigotine-containing patch of the present invention above, independently for each.

Next, the adhesive agent layer composition is spread to obtain the adhesive agent layer. For example, by spreading the adhesive agent layer composition on a surface (usually, one surface) of the backing layer so as to have a desired mass per unit area, and then, if necessary, drying and removing the solvent by heating to form an adhesive agent layer, and further, if necessary, cutting the adhesive agent layer into a desired shape, rotigotine stability can be improved in the resulting rotigotine-containing patch, and the rotigotine-containing patch of the present invention can be obtained.

The method of the present invention may further include a step of bonding the release liner to a surface of the adhesive agent layer opposite to the backing layer, and in this case, the adhesive agent layer composition is first spread on one surface of the release liner so as to have a desired mass per unit area to form an adhesive agent layer, and then the backing layer is bonded to a surface of the adhesive agent layer opposite to the release liner, and if necessary, the laminate is cut into a desired shape to obtain the rotigotine-containing patch.

Further, the resulting patch may be enclosed in a storage packaging container (for example, an aluminum laminate bag) as needed to form a package.

### [Examples]

Hereinafter, the present invention will be described more specifically based on Examples and Comparative Examples, but the present invention is not limited to the following Examples. In each of Examples and Comparative Examples, a skin permeation test and a stability evaluation were performed by the following methods, respectively.

### <Skin Permeation Test (In Vitro Hairless Mouse Skin Permeation Test)>

First, the skin of the torso of a hairless mouse was peeled off and fat was removed to obtain a fat-removed skin piece; then, on the stratum corneum side, a patch that had been cut into a 1.0 cm² square and from which a release liner had been removed was applied to prepare a test sample. The test sample was set in a flow-through diffusion cell so that the dermis side was in contact with a receptor liquid, and the cell was filled with a receptor solution (phosphate buffered saline). Next, while circulating heated circulating water to the outer periphery so that the receptor solution was kept at 32°C, the receptor solution was supplied at a flow rate of about 5 mL/hr, and the receptor solution was collected every 2 hours for 24 hours. The concentration of rotigotine in the collected receptor solution was measured by high performance liquid chromatography, and the amount of rotigotine permeated through the skin per unit area of the adhesive agent layer was calculated by the following formula: Amount of rotigotine permeated through skin (µg/cm2) = {concentration of rotigotine in receptor solution (µg/mL) × flow rate (mL)}/area of patch (cm2), and the amount of permeation per hour (skin permeation rate (µg/cm²/hr)) was determined. The measurement was performed for each of two test samples, and the average value of the maximum values of the respective skin permeation rates within 24 hours was taken as the maximum skin permeation rate (Jmax).

### <Stability Evaluation>

The patches obtained in Examples and Comparative Examples were enclosed in aluminum laminate bags to prepare test samples, and stored at 60°C for 2 weeks. The generation rate [%] of rotigotine degradation products was calculated for the patch after storage by the following method (1) or (2) and used as an index of stability evaluation. Note that the same results can be obtained by the following method (1) and method (2).
(1) First, a release liner was removed from the patch after storage, the adhesive agent layer was immersed in 5 mL of tetrahydrofuran, and a dilution solution (a mixed solution of 0.2% phosphate buffer and acetonitrile (50:50 (v:v))) was added to the resulting solution so that the total volume became 25 mL, and the mixture was shaken and filtered through a filter to prepare a sample solution. Separately, rotigotine at a known concentration was dissolved in the dilution solution to prepare a standard solution.

Using the sample solution, a chromatogram of 7,8-dihydronaphthol and despropyl rotigotine (Despropyl RTN), which are rotigotine degradation products, in the sample solution was obtained by a high performance liquid chromatography apparatus (manufactured by Shimadzu Corporation) under the following conditions:
Column: TSKgel ODS-80Ts QA (4.6 mm I.D. × 150 mm), 5 µm
Mobile phase liquid: mixed solution of 0.2% phosphate buffer containing 10 mM sodium dodecyl sulfate and acetonitrile (50:50 (v:v))
Detection wavelength: 225 nm
Column temperature: 40°C
Flow rate: 0.7 mL/min.

Further, using the standard solution, a chromatogram of rotigotine in the standard solution was obtained by the high performance liquid chromatography apparatus under the same conditions as above. The amount of 7,8-dihydronaphthol generated and the amount of despropyl rotigotine generated were calculated from the area under the curve of rotigotine in the resulting chromatogram and the area under the curve of each of 7,8-dihydronaphthol and despropyl rotigotine in the chromatogram obtained above, respectively. Further, the theoretical content of rotigotine was calculated from the amount of rotigotine blended in the adhesive agent layer, and the generation rate (7,8-dihydronaphthol generation rate [%], Despropyl RTN generation rate [%]) of each rotigotine degradation product in the adhesive agent layer by storing each patch at 60°C for 2 weeks was calculated by dividing each generation amount by the theoretical content. The peak area appearing at around 7.8 minutes was defined as the peak area of 7,8-dihydronaphthol, and the peak area appearing at around 9.7 minutes was defined as the peak area of despropyl rotigotine.

(2)
First, a release liner was removed from the patch after storage, the adhesive agent layer was immersed in 5 mL of tetrahydrofuran, and a dilution solution (a mixed solution of a phosphate buffer (pH 5.5) containing 10 mM dipotassium hydrogen phosphate and 0.067% trimethylamine and acetonitrile (60:40 (v:v))) was added to the resulting solution so that the total volume became 25 mL, and the mixture was shaken and filtered through a filter to prepare a sample solution. Separately, rotigotine at a known concentration was dissolved in the dilution solution to prepare a standard solution.

Using the sample solution, a chromatogram of 7,8-dihydronaphthol and despropyl rotigotine (Despropyl RTN), which are rotigotine degradation products, in the sample solution was obtained by a high performance liquid chromatography apparatus (manufactured by Shimadzu Corporation) under the following conditions:
Column: ZORBAX SB-C18 (4.6 mm I.D. × 250 mm), 5 µm
Mobile phase liquid: phosphate buffer (pH 5.5) (liquid A) containing 10 mM dipotassium hydrogen phosphate and 0.067% trimethylamine, acetonitrile (liquid B); liquid A:liquid B = 90:10 (0 to 20 minutes) → 20:80 (90 to 100 minutes) → 90:10 (105 to 125 minutes)
Detection wavelength: 225 nm
Column temperature: 40°C
Flow rate: 1 mL/min.

Further, using the standard solution, a chromatogram of rotigotine in the standard solution was obtained by the high performance liquid chromatography apparatus under the same conditions as above. The amount of 7,8-dihydronaphthol generated and the amount of despropyl rotigotine generated were calculated from the area under the curve of rotigotine in the resulting chromatogram and the area under the curve of each of 7,8-dihydronaphthol and despropyl rotigotine in the chromatogram obtained above, respectively. Further, the theoretical content of rotigotine was calculated from the amount of rotigotine blended in the adhesive agent layer, and the generation rate (7,8-dihydronaphthol generation rate [%], Despropyl RTN generation rate [%]) of each rotigotine degradation product in the adhesive agent layer by storing each patch at 60°C for 2 weeks was calculated by dividing each generation amount by the theoretical content. The peak area appearing at around 56.8 minutes was defined as the peak area of 7,8-dihydronaphthol, and the peak area appearing at around 44.1 minutes was defined as the peak area of despropyl rotigotine.

### (Example 1)

First, 0.1 parts by mass of propyl gallate was added to 9 parts by mass of rotigotine (free form), 11.73 parts by mass of a styrene-isoprene-styrene block copolymer, 5.03 parts by mass of polyisobutylene, 42.73 parts by mass of an alicyclic saturated hydrocarbon resin, 5 parts by mass of octyldodecanol, 3 parts by mass of cholesterol, 10 parts by mass of crosslinked polyvinylpyrrolidone, and 13.41 parts by mass of liquid paraffin, and the mixture was added to an appropriate amount of a solvent (absolute ethanol and toluene), followed by mixing to obtain an adhesive agent layer composition. Next, the resulting adhesive agent layer composition was spread on a release liner (a polyethylene terephthalate film subjected to release treatment), and the solvent was dried and removed to form an adhesive agent layer having a mass per unit area of 50 g/m². A backing layer (polyethylene terephthalate film) was laminated on the surface of the resulting adhesive agent layer opposite to the release liner to obtain a patch in which the backing layer/the adhesive agent layer/the release liner were laminated in this order.

### (Examples 2 to 4, Comparative Examples 1 to 5)

Patches were obtained in the same manner as in Example 1, except that the compositions of the adhesive agent layer compositions (compositions excluding the solvent) were as shown in Table 1 or Table 2 below.

The patches obtained in Examples 1 to 4 and Comparative Examples 1 to 5 were subjected to a stability evaluation (2 weeks after production, 60°C, Comparative Examples 1 and 2: method (1), Examples 1 to 4 and Comparative Examples 3 to 5: method (2)). The results of the stability evaluation are shown in Tables 1 and 2 together with the compositions (compositions excluding the solvent) of the adhesive agent layer compositions of Examples and Comparative Examples. In each of Examples, it was confirmed by a skin permeation test that a sufficiently excellent maximum skin permeation rate (Jmax) [µg/cm²/hr] was achieved.

**[Table 1]**

| | Example 1 | Example 2 | Example 3 | Example 4 |
|---|---|---|---|---|
| Adhesive agent layer composition [parts by mass] | | | | |
| Rotigotine (free form) | 9 | 9 | 9 | 9 |
| Styrene-isoprene-styrene block copolymer | 11.73 | 11.70 | 11.63 | 11.59 |
| Polyisobutylene | 5.03 | 5.01 | 4.98 | 4.97 |
| Alicyclic saturated hydrocarbon resin | 42.73 | 42.62 | 42.35 | 42.24 |
| Octyldodecanol | 5 | 5 | 5 | 5 |
| Cholesterol | 3 | 3 | 3 | 3 |
| Crosslinked polyvinylpyrrolidone | 10 | 10 | 10 | 10 |
| Liquid paraffin | 13.41 | 13.37 | 13.29 | 13.25 |
| Propyl gallate | 0.1 | 0.3 | 0.1 | 0.3 |
| Sodium thioglycolate | - | - | 0.65 | 0.65 |
| Total | 100 | 100 | 100 | 100 |
| 7,8-Dihydronaphthol generation rate (%) after 2 weeks at 60°C | 0.32 | 0.35 | 0.14 | 0.17 |
| Despropyl RTN generation rate (%) after 2 weeks at 60°C | 0.25 | 0.20 | 0.06 | 0.05 |

**[Table 2]**

| | Comparative Example 1 | Comparative Example 2 | Comparative Example 3 | Comparative Example 4 | Comparative Example 5 |
|---|---|---|---|---|---|
| Adhesive agent layer composition [parts by mass] | | | | | |
| Rotigotine (free form) | 9 | 9 | 9 | 9 | 9 |
| Styrene-isoprene-styrene block copolymer | 12.23 | 12.07 | 12.07 | 12.07 | 12.07 |
| Polyisobutylene | 5.24 | 5.17 | 5.17 | 5.17 | 5.17 |
| Alicyclic saturated hydrocarbon resin | 44.55 | 43.97 | 43.97 | 43.97 | 43.97 |
| Octyldodecanol | 5 | 5 | 5 | 5 | 5 |
| Crosslinked polyvinylpyrrolidone | 10 | 10 | 10 | 10 | 10 |
| Liquid paraffin | 13.98 | 13.79 | 13.79 | 13.79 | 13.79 |
| Propyl gallate | - | - | - | - | - |
| Sodium metabisulfite | - | 1 | - | - | - |
| Ascorbic acid | - | - | 1 | - | - |
| Tocopherol | - | - | - | 1 | - |
| Dibutylhydroxytoluene | - | - | - | - | 1 |
| Total | 100 | 100 | 100 | 100 | 100 |
| 7,8-Dihydronaphthol generation rate (%) after 2 weeks at 60°C | 1.44 | 0.27 | 2.23 | 1.41 | 1.30 |
| Despropyl RTN generation rate (%) after 2 weeks at 60°C | 0.48 | 1.10 | 0.34 | 0.26 | 0.40 |

As is clear from the results shown in Tables 1 and 2, it was confirmed that in the patches comprising propyl gallate in the adhesive agent layer (for example, Examples 1 to 4), the generation rate of rotigotine degradation products was sufficiently suppressed even after 2 weeks of production under the severe condition of a temperature of 60°C, and rotigotine was stabilized at a high level.

On the other hand, in the patches obtained in Comparative Examples 2 to 5, even when components such as sodium metabisulfite conventionally used as drug stabilizers were comprised, at least one of the rotigotine degradation products was generated at a higher rate than in the patches comprising propyl gallate in the adhesive agent layer, and the generation rate of rotigotine degradation products was further increased in some cases (for example, Comparative Examples 2 and 3) as compared with Comparative Example 1 in which no stabilizer was comprised.

### [Industrial Applicability]

As described above, according to the present invention, it is possible to provide a rotigotine-containing patch and a method for improving rotigotine stability, which are particularly excellent in the stability over time of rotigotine and/or a pharmaceutically acceptable salt thereof.

## Claims

1. A rotigotine-containing patch including a backing layer and an adhesive agent layer, wherein the adhesive agent layer comprises an adhesive base agent, propyl gallate, and at least one selected from the group consisting of rotigotine and a pharmaceutically acceptable salt of rotigotine.

2. The rotigotine-containing patch according to claim 1, wherein a content of propyl gallate in the adhesive agent layer is 0.01 to 1% by mass relative to a total mass of the adhesive agent layer.

3. The rotigotine-containing patch according to claim 1 or 2, wherein the adhesive base agent is at least one selected from the group consisting of a rubber-based adhesive base agent, an acrylic-based adhesive base agent, and a silicone-based adhesive base agent.

4. The rotigotine-containing patch according to claim 1 or 2, wherein a content of the at least one selected from the group consisting of rotigotine and a pharmaceutically acceptable salt of rotigotine in the adhesive agent layer is 5 to 15% by mass relative to the total mass of the adhesive agent layer in terms of rotigotine free form.

5. A method for improving stability of at least one selected from the group consisting of rotigotine and a pharmaceutically acceptable salt of rotigotine in a rotigotine-containing patch including a backing layer and an adhesive agent layer, wherein the adhesive agent layer comprises an adhesive base agent and at least one selected from the group consisting of rotigotine and a pharmaceutically acceptable salt of rotigotine, the method including:
a step of further comprising propyl gallate in the adhesive agent layer.

6. The method for improving rotigotine stability according to claim 5, wherein propyl gallate is comprised in the adhesive agent layer such that a content thereof relative to the total mass of the adhesive agent layer is 0.01 to 1% by mass.

7. The method for improving rotigotine stability according to claim 5 or 6, wherein the adhesive base agent is at least one selected from the group consisting of a rubber-based adhesive base agent, an acrylic-based adhesive base agent, and a silicone-based adhesive base agent.

8. The method for improving rotigotine stability according to claim 5 or 6, wherein the content of the at least one selected from the group consisting of rotigotine and a pharmaceutically acceptable salt of rotigotine is 5 to 15% by mass relative to the total mass of the adhesive agent layer in terms of rotigotine free form.
